## Europäisches Patentamt

## European Patent Office

## Office européen des brevets

(19)

(11) Publication number: **0 061 395**
**B1**

# EUROPEAN PATENT SPECIFICATION

(12)

(45) Date of publication of patent specification: **24.10.84**

(21) Application number: **82400486.5**

(22) Date of filing: **17.03.82**

(51) Int. Cl.³: **C 07 C 69/14,** C 07 C 69/16, C 07 C 67/00

(54) **Preparation of ethyl acetate.**

(30) Priority: **17.03.81 US 244572**
**17.03.81 US 244578**

(43) Date of publication of application:
**29.09.82 Bulletin 82/39**

(45) Publication of the grant of the patent:
**24.10.84 Bulletin 84/43**

(84) Designated Contracting States:
**BE DE FR GB IT NL**

(56) References cited:
**EP-A-0 034 062**
**US-A-3 957 827**
**US-A-4 221 918**

(73) Proprietor: **EASTMAN KODAK COMPANY**
**343 State Street**
**Rochester New York 14650 (US)**

(72) Inventor: **Larkins, Thomas Hassell, Jr.**
**Rt.8, 4408 Beechcliff Drive**
**Kingsport Tennessee 37664 (US)**
Inventor: **Tennant, Brent Alan**
**3601 Clearwater Drive**
**Kingsport Tennessee 37664 (US)**
Inventor: **Pond, David Martin**
**1333 Dupont Drive**
**Kingsport Tennessee 37664 (US)**
Inventor: **Glenn, Thomas Jackson**
**5024 Ross Road**
**Kingsport Tennessee 37664 (US)**

(74) Representative: **Parent, Yves et al**
**Kodak-Pathé Département des Brevets et**
**Licences 30, rue des Vignerons B.P. 60**
**F-94302 Vincennes Cedex (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for the preparation of ethyl acetate by hydrogenating acetic anhydride or mixtures of acetic anhydride and ethylidene diacetate.

The hydrogenation of acetic anhydride using a nickel catalyst in the presence of a strong acid, such as hydrochloric acid, hydrofluoric acid, or methane sulfonic acid, is disclosed in U.S. Patent 4,221,918. The products obtained are reported to be ethylidene diacetate, acetic acid and, when hydrochloric acid was the acid used, 1-chloroethyl acetate. When ethyl acetate is the desired product, this prior art process is impractical. The process of this invention provides a very effective method of making ethyl acetate.

In accordance with this invention acetic anhydride or a mixture of acetic anhydride and ethylidene diacetate is hydrogenated with Raney nickel as the hydrogenation catalyst to form ethyl acetate.

The feed to the hydrogenation reactor can, if desired, contain, in addition to acetic anhydride and/or ethylidene diacetate, an inert solvent such as acetic acid. The acetic acid in the product from the reaction can be converted to methyl acetate and used in the production of acetic anhydride.

Acetic anhydride containing little if any ethylidene diacetate, e.g., up to about 10 weight percent, can be readily hydrogenated to produce ethyl acetate in good product yields and space-time yields. However, the presence of larger amounts of ethylidene diacetate inhibits the conversion of acetic anhydride to products and thus, the use of more severe reaction conditions or an organic sulfonic acid is necessary to satisfactorily hydrogenate mixtures of acetic anhydride and ethylidene diacetate to form ethyl acetate.

The concentration of the Raney nickel that is used is in the range of 0.1 to 10 weight percent, based on the acetic anhydride and, when present, ethylidene diacetate. Catalyst concentrations (same basis) of 0.5 to 2.5 weight percent will most often be used.

The temperature for the reaction is in the range of 100 to 250°C, although at the higher temperatures ethyl acetate decomposition can be a problem. Pressures (total reaction pressure) in the range of 500 to 5000 psig (3550 to 34590 kPa) may be used, although the use of pressures in excess of 2500 psig (17340 kPa) normally is not advantageous. The preferred temperatures and pressures vary depending on whether the material being hydrogenated consists primarily of acetic anhydride or consists of a mixture of acetic anhydride and ethylidene diacetate, e.g. mixtures of acetic anhydride and ethylidene diacetate in weight ratios of about 4:1 to 1:4. When the material being hydrogenated consists primarily of acetic anhydride, the preferred temperatures and pressures are in the range of 130 to 200°C, especially 130 to 150°C, and 500 to 1500 psig (3550 to 10445 kPa). When the material is a mixture of acetic anhydride and ethylidene diacetate, preferred temperatures and pressures are 170 to 250°C, especially 200 to 225°C, and 2000 to 2500 psig (13895 to 17340 kPa).

When a substantial amount of ethylidene diacetate is fed to the reaction with the acetic anhydride, for example 50:50 weight mixtures, the ethylidene diacetate resists hydrogenation and retards the hydrogenation of the acetic anhydride. Consequently, relatively severe reaction conditions are required to hydrogenate such mixtures to ethyl acetate when only Raney nickel is used in the reaction. Much milder reaction conditions can be used to hydrogenate such mixtures to ethyl acetate if an organic sulfonic acid is used in the reaction medium. The organic sulfonic acid enables the use of milder, more economical reaction conditions and it results in higher conversions of acetic anhydride and ethylidene diacetate to ethyl acetate.

The amount of organic sulfonic acid that is used is within the range of 0.1 to 3 weight percent, based on the total weight of acetic anhydride and ethylidene diacetate. The preferred amount is 0.5 to 1.5 weight percent. Of the many organic sulfonic acids that can be used, the lower alkyl and aryl sulfonic acids are preferred. The most preferred are methanesulfonic acid and p-toluenesulfonic acid because of their relatively low cost.

When an organic sulfonic acid is used in the hydrogenation process, the reaction temperature can be in the range of 130°C to 175°C, and the reaction pressure can be in the range of 500 to 3000 psig (3550 to 20790 kPa).

The process of the invention may be carried out as a batch operation, or more suitably, as a continuous process wherein acetic anhydride or mixtures of acetic anhydride and ethylidene diacetate are continuously fed to an autoclave and reaction mixture containing the desired products are continuously removed either as liquid or as a vapor. Unreacted materials and coproduct acetic acid may be removed from the reaction mixture after removal from the autoclave by distillation and recycled to the autoclave.

The process of the invention is further illustrated by the following examples.

Examples 1—18

Acetic anhydride (100 g) and mixtures of acetic anhydride (50 g) and ethylidene diacetate (50 g) were hydrogenated in the presence of varying amounts of Raney nickel using different temperatures and total autoclave pressures. The material hydrogenated in Examples 1—7 was acetic anhydride and in Examples 8—18 it was a 50-50 mixture of acetic anhydride and ethylidene diacetate. The acetic anhydride, ethylidene diacetate (when used) and Raney nickel catalyst were loaded into a 300 ml stainless steel, rocking autoclave. The autoclave was purged with 100 psig (739 kPa) hydrogen gas pressure at room temperature and then the gas was vented. The internal pressure of the autoclave was increased to 10 psig (172 kPa) by adding hydrogen gas at room temperature. The autoclave was sealed and heated and rocked until reaction temperature was reached, at which time additional hydrogen gas was added to increase the autoclave internal pressure to the predetermined value. The time at which the autoclave internal pressure reached the predetermined, value was taken as the start of the reaction time. A reaction time of 2 hours was used in Examples 1—16 and 1 hour in Examples 17 and 18. Reaction pressure was maintained at the preset value during the reaction by adding hydrogen gas at the same rate at which it was consumed by the reactants. When the predetermined reaction time was completed the autoclave was cooled by a stream of cold air. After the gas was vented from the autoclave the reaction product was analyzed by gas chromatographic methods.

Table I shows the reaction temperature (°C) and pressure in psig and (kPa), the amount of catalyst (cat., g) charged, the amount (in moles) of ethyl acetate (EA) produced, the percent of acetic anhydride (Ac$_2$O) and ethylidene diacetate (EDA) consumed (cons.) and the product yield (PY, percent of theory) and space-time yields (STY, in grams/liter liquid-hour) for ethyl acetate.

TABLE I

| Ex. | Cat. | Temp. | Press. | | EA | Ac$_2$O Cons. | EDA Cons. | EA PY | EA STY |
|---|---|---|---|---|---|---|---|---|---|
| 1 | 1.5 | 170 | 1000 | (7998) | .42 | 97.8 | — | 87.5 | 185 |
| 2 | 2.5 | 140 | 500 | (3550) | .33 | 78.6 | — | 85.7 | 145 |
| 3 | 0.5 | 140 | 1500 | (10445) | .37 | 80.6 | — | 94.8 | 163 |
| 4 | 2.5 | 140 | 1500 | (10445) | .35 | 89.0 | — | 80.5 | 154 |
| 5 | 0.5 | 200 | 1500 | (10445) | .40 | 99.0 | — | 82.5 | 176 |
| 6 | 2.5 | 200 | 1500 | (10445) | .32 | 100.0 | — | 65.3 | 141 |
| 7 | 0.5 | 140 | 500 | (3550) | .21 | 46.9 | — | 91.3 | 92 |
| 8 | 1.5 | 190 | 1500 | (10445) | .20 | 77.6 | 32.4 | 66.6 | 88 |
| 9 | 2.5 | 170 | 750 | (5275) | .20 | 91.8 | 11.8 | 75.4 | 88 |
| 10 | 0.5 | 210 | 750 | (5275) | .13 | 36.7 | 55.9 | 46.4 | 57 |
| 11 | 0.5 | 170 | 2250 | (15617) | .12 | 46.9 | 8.8 | 82.7 | 53 |
| 12 | 2.5 | 210 | 750 | (5275) | .21 | 67.4 | 76.5 | 49.4 | 92 |
| 13 | 2.5 | 170 | 2250 | (15617) | .25 | 100.0 | 17.6 | 82.0 | 110 |
| 14 | 0.5 | 210 | 2250 | (15617) | .43 | 98.0 | 79.4 | 84.3 | 189 |
| 15 | 2.5 | 210 | 2250 | (15617) | .43 | 100.0 | 85.3 | 80.4 | 189 |
| 16 | 0.5 | 170 | 750 | (5275) | .09 | 26.5 | 50.0 | 38.4 | 40 |
| 17 | 2.0 | 200 | 2000 | (13895) | .35 | 100.0 | 55.0 | 80.8 | 308 |
| 18 | 2.0 | 200 | 2000 | (13895) | .22 | 96.3 | 21.8 | 69.4 | 194 |

Example 19

Using the procedure described for the preceding examples, a mixture of 75 g acetic anhydride and 75 g ethylidene diacetate was hydrogenated for 1 hour at 200°C and 2000 psig (13895 kPa) in the presence of 2.0 g of Raney nickel. Six runs were carried out using the same catalyst. After each run the liquid product was pumped from the autoclave through a metal frit so as to leave the catalyst in the autoclave. Fresh acetic anhydride and ethylidene diacetate were then pumped into the autoclave and the next run was carried out.

Table II shows the amount (moles) of ethyl acetate produced, the percent of the acetic anhydride and ethylidene diacetate consumed and the product yield and spacetime yield for ethyl acetate for each of the six runs. The overall average for each of the results measured is shown at the bottom of the table.

TABLE II

| Run | EA | $Ac_2O$ Cons. | EDA Cons. | EA PY | EA Sty |
|---|---|---|---|---|---|
| 1 | .23 | 100 | 36 | 42 | 165 |
| 2 | .48 | 100 | 59 | 71 | 340 |
| 3 | .29 | 100 | 37 | 53 | 210 |
| 4 | .35 | 100 | 37 | 63 | 253 |
| 5 | .24 | 100 | 38 | 42 | 170 |
| 6 | .36 | 100 | 34 | 66 | 256 |
| Avg. | .33 | 100 | 40 | 56 | 208 |

Examples 20—36

A mixture of acetic anhydride (50 g) and ethylidene diacetate (50 g) was hydrogenated in the presence of varying amounts of Raney nickel (Examples 20—28) and Raney nickel promoted with p-toluenesulfonic acid (Examples 29—36) using different temperatures and total autoclave pressures. The reaction procedure and equipment were identical to Examples 1—18. The reaction time for Examples 20—28 was 2 hours and 1 hour for Examples 29—36.

Table III shows the temperature (°C) and pressure in psig and (kPa), the amount of nickel catalyst (cat., g) and p-toluenesulfonic acid (PTSA, g) charged, the amount (in moles) of ethyl acetate (EA) produced, the percent of acetic anhydride ($Ac_2O$) and ethylidene diacetate (EDA) consumed (cons.) and the Product yield (PY, percent of theory) and space-time yields (STY, in grams/liter liquid-hour) for ethyl acetate.

TABLE III

| Ex. | Cat. | PTSA | Temp. | Press. | EA | Ac$_2$O Cons. | EDA Cons. | EA PY | EA STY |
|---|---|---|---|---|---|---|---|---|---|
| 20 | 1.5 | — | 190 | 1500 (10445) | .20 | 77.6 | 32.4 | 66.6 | 88 |
| 21 | 2.5 | — | 170 | 750 (5275) | .20 | 91.8 | 11.3 | 75.4 | 88 |
| 22 | 0.5 | — | 210 | 750 (5275) | .13 | 36.7 | 55.9 | 46.4 | 57 |
| 23 | 0.5 | — | 170 | 2250 (15617) | .12 | 46.9 | 8.8 | 82.7 | 53 |
| 24 | 2.5 | — | 210 | 750 (5275) | .21 | 67.4 | 76.5 | 49.4 | 92 |
| 25 | 2.5 | — | 170 | 2250 (15617) | .25 | 100.0 | 17.6 | 82.0 | 110 |
| 26 | 0.5 | — | 210 | 2250 (15617) | .43 | 98.0 | 79.4 | 84.3 | 189 |
| 27 | 2.5 | — | 210 | 2250 (15617) | .43 | 100.0 | 85.3 | 80.4 | 189 |
| 28 | 0.5 | — | 170 | 750 (15275) | .09 | 26.5 | 50.0 | 38.4 | 40 |
| 29 | 2.0 | 1.5 | 110 | 1000 (7998) | .08 | 58.2 | 2.4 | 52.5 | 70 |
| 30 | 2.0 | 0.5 | 110 | 2000 (13895) | .11 | 81.6 | 7.1 | 49.1 | 97 |
| 31 | 2.0 | 0.5 | 150 | 1000 (7998) | .31 | 99.6 | 63.8 | 67.1 | 273 |
| 32 | 2.0 | 1.5 | 110 | 2000 (13895) | .10 | 69.4 | 0.3 | 55.6 | 84 |
| 33 | 2.0 | 1.5 | 150 | 1000 (7998) | .39 | 99.8 | 97.4 | 67.0 | 345 |
| 34 | 2.0 | 0.5 | 150 | 2000 (13895) | .28 | 100.0 | 93.5 | 49.7 | 246 |
| 35 | 2.0 | 1.5 | 150 | 2000 (13895) | .43 | 100.0 | 100.0 | 74.2 | 382 |
| 36 | 2.0 | 0.5 | 110 | 1000 (7998) | .09 | 62.9 | 3.5 | 51.8 | 76 |

Table III illustrates the advantages which result from the use of the organic sulfonic acid. In Example 14 almost all of the ethylidene diacetate was consumed whereas in Example 6, using significantly more severe conditions but in the absence of an organic sulfonic acid, only 17.6% of the ethylidene diacetate was consumed.

**Claims**

1. Catalytic process for the preparation of ethyl acetate characterized by hydrogenating acetic anhydride or a mixture of acetic anhydride and ethylidene diacetate by using Raney nickel as a catalyst for the process.

2. The process according to claim 1 wherein the reaction pressure is 500 to 5000 psig (3550 to 34590 kPa) and the reaction temperature is 100° to 250°C.

3. The process according to claim 2 wherein the catalyst concentration is 0.1 to 10 percent, by weight, of acetic anhydride or a mixture of acetic anhydride and ethylidene diacetate.

4. The process according to claim 1 wherein acetic anhydride is hydrogenated at 130°C to 150°C using a catalyst concentration of 0.5 to 2.5 percent, by weight, of acetic anhydride.

5. The process according to claim 1 wherein a mixture of acetic anhydride and ethylidene diacetate is hydrogenated at a pressure of 2000 to 2500 psig (13895 to 17340 kPa) and a temperature of 170 to 250°C.

6. The process according to claim 1 wherein a mixture of acetic anhydride and ethylidene diacetate is hydrogenated at a pressure of 500 to 3000 psig (3550 to 20790 kPa) and at a temperature of 130°C to 175°C and an organic sulfonic acid is used with the Raney nickel in the hydrogenation reaction.

7. The process according to claim 6 wherein the concentration of Raney nickel is 0.1 to 10 percent, by weight, of acetic anhydride and ethylidene diacetate and the concentration of organic sulfonic acid is 0.1 to 3 percent, by weight, of acetic anhydride and ethylidene diacetate.

8. The process according to claim 7 wherein the organic sulfonic acid in methanesulfonic acid or p-toluenesulfonic acid.

9. The process according to claim 8 wherein the pressure is 500 to 1500 psig (3550 to

5

10445 kPa), the temperature is 140°C to 160°C, the concentration of Raney nickel is 0.5 to 2.5 weight percent and the concentration of organic sulfonic acid is 0.5 to 1.5 weight percent.

## Patentansprüche

1. Katalytisches Verfahren zur Herstellung von Ethylacetat, gekennzeichnet durch Hydrieren von Essigsäureanhydrid oder einer Mischung von Essigsäureanhydrid und Ethylidendiacetat unter Verwendung von Raney-Nickel als einen Katalysator für das Verfahren.

2. Verfahren nach Anspruch 1, dadurch gekennzeichnet, daß der Reaktionsdruck bei 3550 bis 34590 kPa und die Reaktionstemperatur bei 100° bis 250°C liegen.

3. Verfahren nach Anspruch 2, dadurch gekennzeichnet, daß die Katalysatorkonzentration bei 0,1 bis 10 Gew.-%, bezogen auf Essigsäureanhydrid oder eine Mischung von Essigsäureanhydrid und Ethylidendiacetat liegt.

4. Verfahren nach Anspruch 1, dadurch gekennzeichnet, daß Essigsäureanhydrid bei 130°C bis 150°C unter Verwendung einer Katalysatorkonzentration von 0,5 bis 2,5 Gew.-%, bezogen auf Essigsäureanhydrid hydriert wird.

5. Verfahren nach Anspruch 1, dadurch gekennzeichnet, daß eine Mischung aus Essigsäureanhydrid und Ethylidendiacetat bei einem Druck von 13895 bis 17340 kPa und einer Temperatur von 170 bis 250°C hydriert wird.

6. Verfahren nach Anspruch 1, dadurch gekennzeichnet, daß eine Mischung aus Essigsäureanhydrid und Ethylidendiacetat bei einem Druck von 3550 bis 20790 kPa und einer Temperatur von 130°C bis 175°C hydriert wird, und daß zur Durchführung der Hydrierungsreaktion mit dem Raney-Nickel eine organische Sulfonsäure verwendet wird.

7. Verfahren nach Anspruch 6, dadurch gekennzeichnet, daß die Konzentration an Raney-Nickel bei 0,1 bis 10 Gew.-%, bezogen auf Essigsäureanhydrid und Ethylidendiacetat, und die Konzentration an organischer Sulfonsäure bei 0,1 bis 3 Gew.-%, bezogen auf Essigsäureanhydrid und Ethylidendiacetat liegt.

8. Verfahren nach Anspruch 7, dadurch gekennzeichnet, daß die organische Sulfonsäure Methansulfonsäure oder p-Toluolsulfonsäure ist.

9. Verfahren nach Anspruch 8, dadurch gekennzeichnet, daß der Druck bei 3550 bis 10445 kPa und die Temperatur bei 140°C bis 160°C liegt und daß die Konzentration an Raney-Nickel 0,5 bis 2,5 Gew.-% und die Konzentration an organischer Sulfonsäure 0,5 bis 1,5 Gew.-% beträgt.

## Revendications

1. Procédé catalytique pour la préparation d'acétate d'éthyle, caractérisé en ce qu'on hydrogène de l'anhydride acétique ou un mélange d'anhydride acétique et de diacétate d'éthylidène en utilisant du nickel de Raney comme catalyseur du procédé.

2. Procédé conforme à la revendication 1, dans lequel la pression de la réaction est de 500 à 5000 psig (3550 à 34 590 kPa) et la température de la réaction est de 100°C à 250°C.

3. Procédé conforme à la revendication 2, dans lequel la concentration du catalyseur est de 0,1 à 10% en masse, par rapport à l'anhydride acétique ou au mélange d'anhydride acétique et de diacétate d'éthylidène.

4. Procédé conforme à la revendication 1, dans lequel on hydrogène l'anhydride acétique à 130°C à 150°C en utilisant une concentration de catalyseur de 0,5 à 2,5% en masse, par rapport à l'anhydride acétique.

5. Procédé conforme à la revendication 1, dans lequel on hydrogène un mélange d'anhydride acétique et de diacétate d'éthylidène à une pression de 2000 à 2500 psig (13895 à 17340 kPa) et à une température de 170°C à 250°C.

6. Procédé conforme à la revendication 1, dans lequel on hydrogène un mélange d'anhydride acétique et de diacétate d'éthylidène à une pression de 500 à 3000 psig (3550 à 20790 kPa) et à une température de 130°C à 175°C et on utilise un acide sulfonique organique avec le nickel de Raney dans la réaction d'hydrogénation.

7. Procédé conforme à la revendication 6, dans lequel la concentration de nickel de Raney est de 0,1 à 10% en masse par rapport à l'anhydride acétique et au diacétate d'éthylidène et la concentration de l'acide sulfonique organique est de 0,1 à 3% en masse, par rapport à l'anhydride acétique et au diacétate d'éthylidène.

8. Procédé conforme à la revendication 7, dans lequel l'acide sulfonique organique est l'acide méthanesulfonique ou l'acide p-toluène-sulfonique.

9. Procédé conforme à la revendication 8, dans lequel la pression est de 500 à 1500 psig (3550 à 10445 kPa), la température est de 140°C à 160°C, la concentration en nickel de Raney est de 0,5 à 2,5% en masse et la concentration en acide sulfonique organique est de 0,5 à 1,5% en masse.